# EUROPÄISCHE PATENTANMELDUNG

(11) **EP 1 203 814 A2**
(43) Veröffentlichungstag der Anmeldung: **08.05.2002**
(21) Anmeldenummer: 01124666.7
(22) Anmeldetag: 16.10.2001
(51) Int. Cl.: C12N 9/88

(54) **Verfahren zur Herstellung eines (R)-Hydroxynitril Lyase Extraktes**

(30) Priorität: 01.11.2000 DE 10054012
(71) Anmelder: Clariant GmbH, 65929 Frankfurt am Main (DE)
(72) Erfinder: Kirschbaum, Bettina, Dr., 65929 Frankfurt (DE); Wilbert, Götz, Dr., 86368 Gersthofen (DE); Effenberger, Franz, Prof. Dr., 70597 Stuttgart (DE); Brand, Stefan Dr., 69198 Schriesheim (DE); Fromm, Andreas, 60326 Frankfurt (DE); Bühler, Holger Dr., 71640 Ludwigsburg (DE)
(74) Vertreter: Clariant Service GmbH

(57) **Zusammenfassung**

Die vorliegende Erfindung betrifft ein Verfahren zur Herstellung eines (R)-Hydroxynitril Lyase-Extraktes durch Extraktion eines (R)-Hydroxynitril Lyase enthaltenden Naturproduktes mit Wasser, in Abwesenheit oder Anwesenheit eines Puffers, bei einem pH-Wert von 3,3 bis 5,5.

## Beschreibung

Die Erfindung betrifft ein Verfahren zur Herstellung eines (R)-Hydroxynitril Lyase-Extraktes.

(R)-Hydroxynitril Lyase katalysiert die enantioselektive Addition von Blausäure an Aldehyde unter Herstellung von optisch aktiven Cyanhydrinen. Optisch aktive Cyanhydrine dienen als Bausteine für die Gewinnung biologisch wirksamer Stoffe, die z. B. in der Pharma- oder Agro-Industrie Einsatz finden (EP 0547655, EP 0276375).

(R)-Hydroxynitril Lyase lässt sich aus verschiedenen natürlichen Quellen gewinnen. Enzyme and Microb. Technol. 1999, 25, 384-391 beschreiben neben entfettetem Mandelmehl, das in großen Mengen einfach zugänglich ist, auch den Einsatz u.a. von Apfelkernen und weiteren weniger leicht erhältlichen Obstkernen bzw. -steinen. Häufig wird das extrahierte Enzym vor dem Einsatz gereinigt, wie in EP-0 547 655 angegeben, oder auf einem polymeren Träger fixiert verwendet, wie in EP-0 276 375 beschrieben. Beide Methoden der Enzymzubereitung sind vergleichsweise aufwendig und dadurch für den industriellen Einsatz wenig attraktiv.

Die natürlichen Enzymquellen lassen sich z.T. auch in situ einsetzen, vgl. Synth. Commun. 1991, 21, 1387-1391. Hier stellt sich bei der technischen Anwendung das Problem, dass größere Mengen HCN-haltiger Feststoffe handzuhaben und zu entsorgen sind.

Der Einsatz von rohem und damit leicht herzustellendem Enzymextrakt ist in Tetrahedron Lett. 1988, 29, 4485 - 4488 beschrieben. Die als Substrate eingesetzten Aldehyde umfassen in der Regel sterisch wenig anspruchsvolle aliphatische Aldehyde, Furfural und Benzaldehyd und dessen 3- bzw. 4-substituierte Derivate. Ausnahmen bilden jedoch 4-Hydroxybenzaldehyd und 3,4-Dihydroxybenzaldehyd, die sich nicht oder nur in geringem Umfang zu den entsprechenden Cyanhydrinen umsetzen lassen, Top. Curr. Chem. 1999, 200, 193-226, insbesondere Table 2 auf Seite 206. 2-substituierte Benzaldehyde finden ebenfalls keinen Einsatz in der enzymkatalysierten, enantioselektiven Cyanhydrinreaktion mit (R)-Hydroxynitril Lyasen, da unter den üblichen Reaktionsbedingungen nur schlechte Umsätze und niedrige ee-Werte erhalten werden.

Im Hinblick auf die vorstehend geschilderten Nachteile besteht ein Bedarf an einem Verfahren zur Herstellung eines (R)-Hydroxynitril Lyase-Extraktes, der eine bessere Aktivität als die gemäß des Standes der Technik hergestellten (R)-Hydroxynitril Lyase-Extrakte aufweist. Das Verfahren soll einfach durchzuführen sein und keinen großen Aufwand bei der technischen Umsetzung erfordern.

Die Aufgabe wird überraschenderweise gelöst durch ein Verfahren zur Herstellung eines (R)-Hydroxynitril Lyase-Extraktes durch Extraktion eines (R)-Hydroxynitril Lyase enthaltenden Naturproduktes mit Wasser, in Abwesenheit oder Anwesenheit eines Puffers, bei einem pH-Wert von 3,3 bis 5,5.

Das erfindungsgemäße Verfahren ist eine einfache und technisch ohne besonderen Aufwand realisierbare Herstellungsmethode für einen (R)-Hydroxynitril Lyase-Extrakt, dessen Aktivität es erlaubt, Aldehyde, die mit bekannten Enzymzubereitungen in optisch aktive Cyanhydrine umgewandelt werden können, vergleichbar oder besser umzusetzen. Darüber hinaus lassen sich überraschenderweise auch aus schwierig umzusetzenden Substraten wie 2-substituierten Benzaldehyden und 4-Hydroxy- bzw. 3,4-Dihydroxybenzaldehyd optisch aktive Cyanhydrine mit guten Umsätzen und hohen ee-Werten herstellen. Hierbei werden für Enzymreaktionen unerwartet hohe Raumzeitausbeuten erhalten. Man setzt als (R)-Hydroxynitril Lyase enthaltendes Naturprodukt mit gutem Erfolg zerkleinerte Fruchtkerne, insbesondere zerkleinerte, entfettete Fruchtkerne ein. Unter Fruchtkernen versteht man Obstkerne und Obststeine.

Nach einer besonders geeigneten Variante setzt man als (R)-Hydroxynitril Lyase enthaltendes Naturprodukt zerkleinerte, entfettete Apfel- oder Mandelkerne ein. Es lassen sich jedoch auch andere (R)-Hydroxynitril Lyase enthaltende Obstkerne oder Obststeine, die vorzugsweise zerkleinert und entfettet sind, in das erfindungsgemäße Verfahren einsetzen.

Das erfindungsgemäße Verfahren verwendet nach einer bevorzugten Variante entfettetes Mandelmehl, so wie es bei der Herstellung von Mandelöl als Abfallprodukt anfällt.

Die Enzymextraktion wird bei einer Temperatur von 0 bis 60°C, insbesondere 10 bis 50°C, bevorzugt 20 bis 40°C durchgeführt.

Wie vorstehend bereits erwähnt, wird die Extraktion des (R)-Hydroxynitril Lyase enthaltenden Naturproduktes mit Wasser in Abwesenheit oder Anwesenheit eines Puffers durchgeführt. Arbeitet man ohne Puffer, so muss man Sorge dafür tragen, dass der vorgegebene pH-Wert von 3,3 bis 5,5 während der gesamten Extraktion eingehalten wird. Dies erreicht man, da während der Extraktion der pH-Wert auf Werte > 5,5 ansteigt, durch eine kontrollierte Zugabe von Säure, beispielsweise einer Mineralsäure, während der Extraktion. Das für die Extraktion verwendete Wasser wird zuvor auch durch Säurezugabe auf einen entsprechenden pH-Wert eingestellt. Eine derartige Arbeitsweise ist Beispiel 5 zu entnehmen. pH-Werte oberhalb 5,5 führen zu Extrakten mit verringerter Wirkung in der enantioselektiven Cyanhydrinreaktion, siehe Beispiel 4a (Vergleichsbeispiel).

Man setzt üblicherweise das (R)-Hydroxynitril Lyase enthaltende Naturprodukt und Wasser bzw. wässrige Pufferlösung im Gewichtsverhältnis 1 : (1 bis 50), insbesondere 1 : (2 bis 20), bevorzugt 1 : (2,5 bis 10) ein.

In einer Reihe von Fällen kann es von Vorteil sein, die Extraktion des (R)-Hydroxynitril Lyase enthaltenden Naturproduktes in Anwesenheit eines Puffers durchzuführen. Besonders geeignet sind Puffer bzw. Puffergemische, die ihre Pufferwirkung in dem angegebenen pH-Bereich von 3,3 bis 5,5 entfalten und den pH-Wert während der Extraktion in diesem Bereich halten. Reicht die puffernde Wirkung des Puffers nicht aus, den pH-Wert während der Extraktion in dem vorgegebenen Bereich zu halten, muss ebenfalls durch Säurezugabe der pH-Wert eingestellt werden.

Geeignete Puffer sind beispielsweise Glutaminsäure-Glutamat-, Phosphorsäure-Phosphat-, Essigsäure-Acetat- und Citronensäure-Citrat-Puffer, insbesondere Essigsäure-Acetat- und Citronensäure-Citrat-Puffer.

Es hat sich als nützlich erwiesen, die Extraktion in Anwesenheit von 20 bis 500 mmol Puffer/Liter, insbesondere 40 bis 300 mmol Puffer/Liter, bevorzugt 80 bis 160 mmol Puffer/Liter durchzuführen. Üblicherweise löst man den Puffer in Wasser und setzt ihn in Form einer 20 bis 500, insbesondere 40 bis 300, bevorzugt 80 bis 160 mmol Puffer je Liter enthaltenden wässrigen Lösung ein.

Die Extraktion gestaltet sich besonders einfach, wenn der pH-Wert des Puffers und die Menge des Puffers so gewählt werden, dass sichergestellt ist, dass der vorgegebene pH-Bereich während der gesamten Extraktion eingehalten wird.

Das Naturprodukt kann auch in nicht entfetteter Form eingesetzt werden. Hierbei ist dessen Anteil im Verhältnis zur Pufferlösung entsprechend dem Fettgehalt zu erhöhen.

Nach einer angemessenen Einwirkdauer des Wassers bzw. der Pufferlösung auf das Naturprodukt von beispielsweise 0,5 bis 24, insbesondere 2 bis 20, bevorzugt 3 bis 18 Stunden, wird der Enzymextrakt üblicherweise durch Filtration mit einem geeigneten Filterapparat vom Naturprodukt getrennt.

Man kann den wässrigen Enzymextrakt zusammen mit dem extrahierten Naturprodukt für die enantioselektive HCN-Addition verwenden. Es ist jedoch günstig, das extrahierte Naturprodukt abzutrennen und den wässrigen, vom Naturprodukt befreiten Enzymextrakt zu benutzen. Auf diese Weise vermeidet man durch HCN verunreinigtes Naturprodukt als Abfall.

Vorteilhafterweise ist eine Umsetzung mit einem der Erfindung entsprechenden Enzymextrakt und einem Substrat, beispielsweise einem aromatischen Aldehyd, in einem Zweiphasensystem durchzuführen, wobei das Substrat in einem organischen, mit Wasser nicht mischbaren Lösungsmittel, in Anwesenheit von HCN, eingesetzt wird.

Bei der Reaktion unter Einsatz des erfindungsgemäßen Enzymextraktes muss auf eine ausreichende Durchmischung geachtet werden.

Der wässrige Enzymextrakt kann nach Beendigung der Reaktion von der organischen, cyanhydrinhaltigen Phase abgetrennt und in eine Folgereaktion eingesetzt werden.

Die Erfindung wird durch die nachfolgenden Beispiele näher erläutert, ohne durch sie eingeschränkt zu werden.

Die Aktivitäten der hergestellten Enzymextrakte wurden nach einer Methode von M. Bauer, H. Griengl und W. Steiner Biotechnol. Bioeng. 1999, 62, 23 bestimmt.

Die ee-Werte der erhaltenen Cyanhydrine wurden nach Derivatisierung mit Acetanhydrid/Pyridin gaschromatographisch über eine β-Cyclodextrin-Säule bestimmt. (Definition 1 Unit siehe auch K. Drauz, H. Waldmann Enzyme Catalysis in Organic Synthesis, Vol. I, Verlag Chemie, Weinheim, 1995, S. 22.)

### Experimenteller Teil:

Unter Enzym wird nachfolgend stets (R)-Hydroxynitril Lyase verstanden.

### Beispiel 1:

### Herstellung von Enzymextrakt mit 80 mmol Citrat-Puffer/Liter, pH 4,8

8,4 g Citronensäure-Monohydrat werden mit demineralisiertem Wasser auf 500 ml aufgefüllt. Mit wenigen Tropfen 50 % NaOH-Lösung wird der pH auf 4,8 eingestellt. 100 g entfettetes Mandelmehl werden mit 500 ml dieses Citrat-Puffers versetzt und 16 Stunden bei Raumtemperatur gerührt. Es wird über eine Glasfritte filtriert. Man erhält etwa 400 ml wässrigen Enzymextrakt, mit einem pH von 5,2 und einer Aktivität von etwa 200 U/ml.

### Herstellung von (R)-2-Chlorbenzaldehydcyanhydrin

56,2 g 2-Chlorbenzaldehyd (0,4 mol) werden in 200 ml Diisopropylether gelöst und mit 200 ml des vorstehend hergestellten Enzymextraktes (40000 U) und 16,2 g HCN (0,6 mol) versetzt. Die Reaktionsmischung wird 45 min bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-2-Chlorbenzaldehydcyanhydrin (Umsatz laut GC 99 %), mit einem ee von 83 %.

### Beispiel 2:

### Herstellung von Enzymextrakt mit 80 mmol Citrat-Puffer/Liter, pH 3,3

8,4 g Citronensäure-Monohydrat werden mit demineralisiertem Wasser auf 500 ml aufgefüllt. Mit wenigen Tropfen 50 % NaOH-Lösung wird der pH auf 3,3 eingestellt. 100 g entfettetes Mandelmehl werden mit 500 ml dieses Citrat-Puffers versetzt und 16 Stunden bei Raumtemperatur gerührt. Es wird über eine Glasfritte filtriert. Man erhält etwa 400 ml wässrigen Enzymextrakt, mit einem pH von 4,4 und einer Aktivität von etwa 75 U/ml.

### Herstellung von (R)-2-Chlorbenzaldehydcyanhydrin

28,1 g 2-Chlorbenzaldehyd (0,2 mol) werden in 100 ml Diisopropylether gelöst und mit 270 ml des vorstehend hergestellten Enzymextraktes (20000 U) und 8,1 g HCN (0,3 mol) versetzt. Die Reaktionsmischung wird 90 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-2-Chlorbenzaldehydcyanhydrin (Umsatz laut GC 95 %), mit einem ee von 82 %.

### Beispiel 3:

### Herstellung von Enzymextrakt mit 160 mmol Citrat-Puffer/Liter, pH 4,8

16,8 g Citronensäure-Monohydrat werden mit demineralisiertem Wasser auf 500 ml aufgefüllt. Mit wenigen Tropfen 50 % NaOH-Lösung wird der pH auf 4,8 eingestellt. 100 g entfettetes Mandelmehl werden mit 500 ml dieses Citrat-Puffers versetzt und 16 Stunden bei Raumtemperatur gerührt. Es wird über eine Glasfritte filtriert. Man erhält etwa 400 ml wässrigen Enzymextrakt, mit einem pH von 5,0 und einer Aktivität von etwa 200 U/ml.

### Herstellung von (R)-2-Chlorbenzaldehydcyanhydrin

28,1 g 2-Chlorbenzaldehyd (0,2 mol) werden in 100 ml Diisopropylether gelöst und mit 100 ml des vorstehend hergestellten Enzymextraktes (20000 U) und 8,1 g HCN (0,3 mol) versetzt. Die Reaktionsmischung wird 45 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-2-Chlorbenzaldehydcyanhydrin (Umsatz laut GC 98 %), mit einem ee von 83 %.

### Beispiel 4:

### Herstellung von Enzymextrakt mit 20 mmol Citrat-Puffer/Liter, pH 3,3

2,1 g Citronensäure-Monohydrat werden mit demineralisiertem Wasser auf 500 ml aufgefüllt. Mit wenigen Tropfen 50 % NaOH-Lösung wird der pH auf 3,3 eingestellt. 100 g entfettetes Mandelmehl werden mit 500 ml dieses Citrat-Puffers versetzt und 16 Stunden bei Raumtemperatur gerührt. Es wird über eine Glasfritte filtriert. Man erhält etwa 400 ml wässrigen Enzymextrakt, mit einem pH von 5,0 und einer Aktivität von etwa 70 U/ml.

### Herstellung von (R)-2-Chlorbenzaldehydcyanhydrin

28,1 g 2-Chlorbenzaldehyd (0,2 mol) werden in 100 ml Diisopropylether gelöst und mit 285 ml des vorstehend hergestellten Enzymextraktes (20000 U) und 8,1 g HCN (0,3 mol) versetzt. Die Reaktionsmischung wird 60 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-2-Chlorbenzaldehydcyanhydrin (Umsatz laut GC 98 %), mit einem ee von 83 %.

### Beispiel 4a (Vergleichsbeispiel):

Herstellung von Enzymextrakt mit 20 mmol Citrat-Puffer/Liter, pH 5,5 (in Anlehnung an das in Synth. Commun. 1991, 21, Seite 1388 beschriebene Verfahren, jedoch mittels Extraktion und Abtrennung von Mandelmehl)

2,1 g Citronensäure-Monohydrat werden mit demineralisiertem Wasser auf 500 ml aufgefüllt. Mit wenigen Tropfen 50 % NaOH-Lösung wird der pH auf 5,5 eingestellt. 100 g entfettetes Mandelmehl werden mit 500 ml dieses Citrat-Puffers versetzt und 16 Stunden bei Raumtemperatur gerührt. Der pH-Wert steigt während der Extraktion - wie nachfolgend bei dem erhaltenen Enzymextrakt ausgewiesen - deutlich an. Es wird über eine Glasfritte filtriert. Man erhält etwa 400 ml wässrigen Enzymextrakt, mit einem pH von 6,0 und einer Aktivität von etwa 70 U/ml.

### Herstellung von (R)-2-Chlorbenzaldehydcyanhydrin

28,1 g 2-Chlorbenzaldehyd (0,2 mol) werden in 100 ml Diisopropylether gelöst und mit 285 ml des vorstehend hergestellten Enzymextraktes (20000 U) und 8,1 g HCN (0,3 mol) versetzt. Die Reaktionsmischung wird 60 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die organische Phase enthält (R)-2-Chlorbenzaldehydcyanhydrin (Umsatz laut GC 99 %), mit einem ee von 55 %.

Die Wirkung des Enzymextraktes ist, wie ein einfacher Vergleich der ee-Werte (ee = enantiomeric excess) bei der Herstellung von (R)-2-Chlorbenzaldehydcyanhydrin in Beispiel 4 deutlich macht, erheblich geringer als bei Verwendung eines erfindungsgemäß hergestellten Enzymextrakts.

### Beispiel 5:

Herstellung von Enzymextrakt mit wässriger Lösung, pH 4,5 - 5,2 (ohne Puffer) 500 ml demineralisiertes Wasser werden mit wenigen ml konz. HCI auf pH 4,5 eingestellt. 100 g entfettetes Mandelmehl werden mit dieser Lösung versetzt und 16 Stunden bei Raumtemperatur gerührt, wobei der pH-Wert mittels kontinuierlicher Zugabe von konz. HCI in einem pH-Bereich von 4,5 - 5,2 gehalten wird. Es wird über eine Glasfritte filtriert. Man erhält etwa 400 ml wässrigen Enzymextrakt, mit einem pH von 4,8 und einer Aktivität von etwa 200 U/ml.

### Herstellung von (R)-2-Chlorbenzaldehydcyanhydrin

28,1 g 2-Chlorbenzaldehyd (0,2 mol) werden in 100 ml Diisopropylether gelöst und mit 100 ml des vorstehend hergestellten Enzymextraktes (20000 U) und 8,1 g HCN (0,3 mol) versetzt. Die Reaktionsmischung wird 60 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-2-Chlorbenzaldehydcyanhydrin (Umsatz laut GC 99 %), mit einem ee von 83 %.

### Beispiel 6:

### Herstellung von Enzymextrakt mit 80 mmol Citrat-Puffer/Liter, pH 4,8

Die Herstellung des Enzymextraktes erfolgt wie in Beispiel 1 beschrieben.

### Herstellung von (R)-Benzaldehydcyanhydrin

74,3 g Benzaldehyd (0,7 mol) werden in 250 ml Diisopropylether gelöst und mit 100 ml des vorstehend genannten Enzymextraktes (20000 U) und 27 g HCN (1,0 mol) versetzt. Die Reaktionsmischung wird 180 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-Benzaldehydcyanhydrin (Umsatz laut GC quant.), mit einem ee von 98 %.

### Beispiel 7:

### Herstellung von Enzymextrakt mit 80 mmol Citrat-Puffer/Liter, pH 4.8

Die Herstellung des Enzymextraktes erfolgt wie in Beispiel 1 beschrieben.

### Herstellung von (R)-3-Hydroxybenzaldehydcyanhydrin

12,2 g 3-Hydroxybenzaldehyd (0,1 mol) werden in 100 ml Diisopropylether gelöst und mit 100 ml des vorstehend genannten Enzymextraktes (20000 U) und 4 g HCN (0,15 mol) versetzt. Die Reaktionsmischung wird 105 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-3-Hydroxybenzaldehydcyanhydrin (Umsatz laut GC 96 %), mit einem ee von 97 %.

### Beispiel 8:

### Herstellung von Enzymextrakt mit 80 mmol Citrat-Puffer/Liter, pH 4,8

Die Herstellung des Enzymextraktes erfolgt wie in Beispiel 1 beschrieben.

### Herstellung von (R)-3-Hydroxybenzaldehydcyanhydrin

48,8 g 3-Hydroxybenzaldehyd (0,4 mol) werden in 200 ml Diisopropylether gelöst und mit 100 ml des vorstehend genannten Enzymextraktes (20000 U) und 16 g HCN (0,6 mol) versetzt. Die Reaktionsmischung wird 225 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-3-Hydroxybenzaldehydcyanhydrin (Umsatz laut GC 94 %), mit einem ee von 92 %.

### Beispiel 9:

### Herstellung von Enzymextrakt mit 80 mmol Citrat-Puffer/Liter, pH 4,8

Die Herstellung des Enzymextraktes erfolgt wie in Beispiel 1 beschrieben.

### Herstellung von (R)-4-Hydroxybenzaldehydcyanhydrin

12,2 g 4-Hydroxybenzaldehyd (0,1 mol) werden in 100 ml Diisopropylether gelöst und mit 100 ml des vorstehend genannten Enzymextraktes (20000 U) und 4 g HCN (0,15 mol) versetzt. Die Reaktionsmischung wird 165 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-4-Hydroxybenzaldehydcyanhydrin (Umsatz laut GC 70 %), mit einem ee von 92 %.

### Beispiel 9a (Vergleichsbeispiel):

### Herstellung von Enzymextrakt mit 80 mmol Citrat-Puffer/Liter, pH 5,3

8,4 g Citronensäure-Monohydrat werden mit demineralisiertem Wasser auf 500 ml aufgefüllt. Mit wenigen Tropfen 50 % NaOH-Lösung wird der pH auf 5,3 eingestellt. 100 g entfettetes Mandelmehl werden mit 500 ml Citrat-Puffer versetzt und 16 Stunden bei Raumtemperatur gerührt. Es wird über eine Glasfritte filtriert. Man erhält etwa 400 ml wässrigen Enzymextrakt, mit einem pH von 5,7 und einer Aktivität von etwa 200 U/ml.

### Herstellung von (R)-4-Hydroxybenzaldehydcyanhydrin

12,2 g 4-Hydroxybenzaldehyd (0,1 mol) werden in 100 ml Diisopropylether gelöst und mit 100 ml des vorstehend hergestellten Enzymextraktes (20000 U) und 4 g HCN (0,15 mol) versetzt. Die Reaktionsmischung wird 165 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die organische Phase enthält (R)-4-Hydroxybenzaldehydcyanhydrin (Umsatz laut GC 63 %), mit einem ee von 70 %.

### Beispiel 10:

### Herstellung von Enzymextrakt mit 80 mmol Citrat-Puffer/Liter, pH 4,8

Die Herstellung des Enzymextraktes erfolgt wie in Beispiel 1 beschrieben.

### Herstellung von (R)-3,4-Dihydroxybenzaldehydcyanhydrin

6,9 g 3,4-Dihydroxybenzaldehyd (0,05 mol) werden in 100 ml Diisopropylether gelöst und mit 100 ml des vorstehend genannten Enzymextraktes (20000 U) und 3 g HCN (0,1 mol) versetzt. Die Reaktionsmischung wird 165 Minuten bei Raumtemperatur heftig gerührt, wobei eine Emulsion entsteht. Nach dem Beenden des Rührens werden etwa 70 % des ursprünglich eingesetzten wässrigen Enzymextraktes abgetrennt. Die Verwendung dieses Extraktes für weitere Reaktionen ist möglich. Die organische Phase enthält (R)-3,4-Dihydroxybenzaldehydcyanhydrin (Umsatz laut GC 65 %), mit einem ee von 76 %.

## Patentansprüche

1. Verfahren zur Herstellung eines (R)-Hydroxynitril Lyase-Extraktes durch Extraktion eines (R)-Hydroxynitril Lyase enthaltenden Naturproduktes mit Wasser, in Abwesenheit oder Anwesenheit eines Puffers, bei einem pH-Wert von 3,3 bis 5,5.

2. Verfahren nach Anspruch 1, **dadurch gekennzeichnet, dass** man als (R)-Hydroxynitril Lyase enthaltendes Naturprodukt zerkleinerte Fruchtkerne einsetzt.

3. Verfahren nach Anspruch 1 oder 2, **dadurch gekennzeichnet, dass** man als (R)-Hydroxynitril Lyase enthaltendes Naturprodukt zerkleinerte, entfettete Fruchtkerne einsetzt.

4. Verfahren nach einem oder mehreren der Ansprüche 1 bis 3, **dadurch gekennzeichnet, dass** man als (R)-Hydroxynitril Lyase enthaltendes Naturprodukt zerkleinerte, entfettete Apfel- oder Mandelkerne einsetzt.

5. Verfahren nach einem oder mehreren der Ansprüche 1 bis 4, **dadurch gekennzeichnet, dass** man in Anwesenheit von 20 bis 500 mmol Puffer/Liter extrahiert.

6. Verfahren nach einem oder mehreren der Ansprüche 1 - 5, **dadurch gekennzeichnet, dass** man in Anwesenheit von 40 bis 300 mmol Puffer/Liter extrahiert.

7. Verfahren nach einem oder mehreren der Ansprüche 1 - 6, **dadurch gekennzeichnet, dass** man in Anwesenheit von 80 bis 160 mmol Puffer/Liter extrahiert.

8. Verfahren nach einem oder mehreren der Ansprüche 1 bis 7, **dadurch gekennzeichnet, dass** man in Anwesenheit eines Citronensäure-Citrat-Puffers extrahiert.

9. Verfahren nach einem oder mehreren der Ansprüche 1 bis 8, **dadurch gekennzeichnet, dass** man bei einem pH-Wert von 4,0 bis 5,5 extrahiert.

10. Verfahren nach einem oder mehreren der Ansprüche 1 bis 9, **dadurch gekennzeichnet, dass** man bei einem pH-Wert von 4,5 bis 5,3 extrahiert.
